# FASCICULE DE BREVET EUROPEEN

(11) **EP 3 958 754 B1**
(45) Date de publication et mention de la délivrance du brevet: **03.04.2024**
(21) Numéro de dépôt: 20742439.1
(22) Date de dépôt: 24.04.2020
(51) Int. Cl.: A61B 17/32, A61B 17/34, A61B 1/31, A61B 5/00, A61B 1/00, A61B 17/00

(54) **DISPOSITIF POUR LE TRAITEMENT DE LA MALADIE HEMORROÏDAIRE**
VORRICHTUNG ZUR BEHANDLUNG VON HÄMORRHOIDEN
DEVICE FOR THE TREATMENT OF HAEMORRHOIDAL DISEASE

(30) Priorité: 26.04.2019 FR 1904457
(43) Date de publication de la demande: 02.03.2022
(73) Titulaire: Odet, Emmanuel, 71870 Hurigny (FR)
(72) Inventeur: Odet, Emmanuel, 71870 Hurigny (FR)
(74) Mandataire: Cabinet Chaillot
(86) Numéro de dépôt international: PCT/IB2020/053894
(87) Numéro de publication internationale: WO 2020/217223

(56) Documents cités:
- EP-A1- 2 818 097
- WO-A1-2006/116779
- US-A- 457 787
- US-A1- 2014 200 404
- US-B2- 8 679 007

## Description

La présente invention se rapporte au domaine médical de la maladie hémorroïdaire, et porte en particulier sur un dispositif pour le traitement de cette maladie.

Dans sa forme préférée, la présente invention propose un dispositif approprié pour la détection, le traitement et la surveillance post-opératoire de la maladie hémorroïdaire dans ses différents stades, ainsi que le traitement du prolapsus hémorroïdaire par mucopexie.

L'une des méthodes de traitement de la maladie hémorroïdaire est la technique de ligature des artères hémorroïdaires sous contrôle Doppler, avec mucopexie en cas de prolapsus hémorroïdaire (HAL-RAR Doppler).

On peut par exemple se référer à la demande internationale PCT WO 03/022155 A1, qui décrit un dispositif classique permettant de mettre en oeuvre la technique HAL Doppler. Ce dispositif comprend un corps tubulaire cylindrique dont l'extrémité distale est fermée en forme d'ogive et dont l'extrémité proximale est ouverte et à partir de laquelle s'étend une partie de préhension par laquelle le chirurgien peut manipuler le corps du dispositif. Une encoche est ménagée dans la paroi latérale du corps et forme une fenêtre par laquelle le chirurgien peut accéder au niveau du bas rectum, et le corps porte une sonde Doppler, au niveau de l'encoche ou entre l'encoche et l'extrémité proximale, afin de détecter les artères hémorroïdaires.

En pratique, pour traiter les hémorroïdes, le chirurgien introduit le corps dans le canal anal du patient sous anesthésie, jusqu'à ce que l'encoche soit au niveau du bas rectum, au contact de la muqueuse rectale. Ensuite, le chirurgien tourne lentement le dispositif autour de l'axe longitudinal dudit corps, jusqu'à ce la sonde Doppler détecte une première artère hémorroïdaire. Une onde sera alors indiquée sur le dispositif d'affichage auquel la sonde Doppler est reliée. Une fois que la première artère hémorroïdaire est détectée, le chirurgien procède à la ligature de celle-ci, via ladite fenêtre, à l'aide d'un ligateur qu'il introduit dans l'intérieur du corps tubulaire. Après la ligature de la première artère hémorroïdaire, le chirurgien recommence à faire tourner lentement le dispositif jusqu'à détection d'éventuelle(s) autre(s) artère(s) hémorroïdaire(s), et procède à leur ligature de la même manière.

Par sécurité, après que le dispositif a fait un tour complet, le chirurgien doit déplacer le dispositif vers l'orifice anal, sur une petite distance, puis doit de nouveau faire tourner lentement le corps pour détecter une ou plusieurs artères hémorroïdaires qui n'auraient pas été repérées lors du premier tour complet.

Après le second tour complet, le chirurgien retire entièrement le dispositif et, en cas de prolapsus hémorroïdaire, procède à une ou plusieurs mucopexies.

Bien qu'en principe le dispositif doit permettre de mettre en oeuvre de manière satisfaisante la technique de ligature des artères hémorroïdaires sous contrôle Doppler, il existe en pratique un risque qu'une ou plusieurs artères hémorroïdaires ne soient pas détectées.

Selon l'inventeur, chirurgien proctologue, de la présente invention, ce risque est principalement dû au fait que le chirurgien doit déplacer l'ensemble du dispositif le long du canal anal et qu'il est bien souvent assez délicat pour le chirurgien de maîtriser parfaitement la profondeur à laquelle l'encoche se situe le long du canal anal.

De plus, la fenêtre d'accès que forme l'encoche est relativement petite, ce qui ne facilite pas les opérations de ligature.

Le présent inventeur a recherché une solution à ce problème, et donc à proposer un dispositif avec lequel le risque de non-détection d'une artère hémorroïdaire est réduit et les opérations de ligature sont facilitées. Selon la présente invention, ce problème peut être résolu par le fait de prévoir un obturateur mobile qui obture une ouverture latérale du corps et qui est déplaçable le long du corps, faisant ainsi varier la longueur de ladite ouverture. De cette manière, ce n'est plus l'ensemble du dispositif qui est déplacé après le premier tour complet, mais uniquement l'obturateur mobile dont le déplacement est bien plus aisé à maîtriser. De plus, après déplacement de l'obturateur mobile à l'opposé de l'extrémité distale du corps, la fenêtre d'accès est agrandie et il est donc plus aisé pour le chirurgien de procéder aux ligatures.

Le présent inventeur s'est aperçu que ce principe d'obturateur mobile n'est pas limité à une utilisation au bloc opératoire, à savoir pendant le traitement chirurgical, mais pourrait également être mis en oeuvre dans un dispositif dont le corps a un diamètre plus petit pour une utilisation en consultation préopératoire ou post-opératoire, au cours de laquelle le dispositif permet au chirurgien d'observer l'intérieur de l'anus et de détecter le réseau artériel hémorroïdaire.

En ce sens, le dispositif selon la présente invention permet un parcours de soin pré-, per- et post-opératoire pour la maladie hémorroïdaire.

Le brevet américain US 457 787 A divulgue un dispositif pour le traitement de la maladie hémorroïdaire tel que défini dans le préambule de la revendication 1.

La présente invention a donc pour objet un dispositif pour le traitement de la maladie hémorroïdaire, comprenant :
- un corps tubulaire cylindrique destiné à être partiellement introduit dans l'anus d'un patient, l'extrémité distale du corps étant fermée tandis que son extrémité proximale est ouverte pour autoriser un accès à l'intérieur du corps, une ouverture latérale étant ménagée dans la paroi latérale du corps de façon à former une fenêtre d'accès au bas rectum,
- des moyens de préhension s'étendant à partir de l'extrémité proximale du corps, pour permettre la manipulation du corps depuis l'extérieur une fois le corps introduit dans l'anus du patient,
- un obturateur mobile obturant en partie l'ouverture latérale du corps de façon à délimiter avec cette dernière la fenêtre d'accès, l'obturateur mobile étant déplaçable en translation le long du corps, suivant l'axe longitudinal de ce dernier, de façon à faire varier la longueur de la fenêtre d'accès, et
- des moyens d'arrêt en translation de l'obturateur mobile par rapport au corps, aptes à arrêter de manière sélective l'obturateur mobile au moins à une première profondeur et une deuxième profondeur le long du corps, la première profondeur étant la position où l'obturateur mobile est le plus proche de l'extrémité distale du corps et où ladite fenêtre d'accès permet, en utilisation, un accès aux artères au niveau du bas du rectum,
caractérisé par le fait que les moyens d'arrêt en translation sont également aptes à arrêter de manière sélective l'obturateur mobile à une troisième profondeur, à distance de la deuxième profondeur suivant l'axe longitudinal du corps.

De préférence, la deuxième profondeur est à 10 mm, suivant l'axe longitudinal du corps, de la première profondeur.

Les moyens d'arrêt en translation définissent ainsi, par rapport à la position du premier tour complet, la position à laquelle le deuxième tour complet de sécurité est effectué, de sorte que le chirurgien n'a plus à se préoccuper de savoir s'il a effectivement déplacé l'obturateur mobile de la distance adéquate. En pratique, une distance de 10 mm entre le plan dans lequel est effectué le premier tour complet et le second plan est la distance optimale. Celle-ci reste à distance acceptable de la ligne pectinée pour ne pas générer de douleurs. Le chirurgien s'assure alors d'avoir détecté les artères hémorroïdaires restantes.

De façon encore davantage préférée, la troisième profondeur est à 10 mm de la deuxième profondeur.

Cet arrêt de l'obturateur à une troisième profondeur permet d'obtenir une fenêtre de taille suffisante et correctement positionnée pour que le chirurgien puisse réaliser la mucopexie d'un prolapsus hémorroïdaire sans avoir à retirer le dispositif hors du patient, le dispositif empêchant l'anus de se refermer et permettant de bien voir et éviter la ligne pectinée. Les trois étapes du traitement que sont la détection des artères hémorroïdaires, la ligature de celles-ci et la mucopexie des éventuels prolapsus hémorroïdaires, peuvent ainsi être réalisées sans déplacement du corps le long du canal anal après que la première artère hémorroïdaire a été détectée.

De préférence, à la première profondeur, l'extrémité distale de l'obturateur mobile est à une distance, suivant l'axe longitudinal du corps, de 55 mm de l'extrémité proximale du corps. La fenêtre d'accès se trouve alors à la position optimale pour la première rotation du dispositif jusqu'à la détection de la première artère hémorroïdaire.

Selon un mode de réalisation particulier, l'obturateur mobile est monté coulissant dans l'ouverture latérale du corps.

Selon un mode de réalisation particulier, les moyens d'arrêt en translation comprennent, d'une part, des crans ménagés sur l'un parmi les bords longitudinaux de l'ouverture latérale du corps et les bords longitudinaux de l'obturateur mobile, et, d'autre part, des organes d'arrêt prévus sur l'autre parmi les bords longitudinaux de l'ouverture latérale du corps et les bords longitudinaux de l'obturateur mobile, les crans et les organes d'arrêt étant positionnés pour qu'à chacune des première, deuxième et troisième profondeurs, au moins un organe d'arrêt est engagé dans un cran.

Ainsi, chacune des profondeurs correspond à un engagement d'au moins un organe d'arrêt dans un cran respectif. L'obturateur mobile peut comprendre deux paires de crans, de préférence trois paires de crans, chaque cran d'une même paire étant situé sur un bord longitudinal respectif de l'obturateur mobile, avec de préférence un espacement de 10 mm, suivant l'axe longitudinal du corps, entre deux paires de crans successives, et le corps comprend des paires d'organes d'arrêt en même nombre que les paires de crans, chaque organe d'arrêt d'une même paire étant situé sur un bord longitudinal respectif de l'ouverture latérale, et l'espacement entre deux paires d'organes d'arrêt successives étant égal à l'espacement entre deux paires de crans successives.

On prévoira deux paires de crans, et donc deux paires d'organes d'arrêt, lorsque les moyens d'arrêt sont configurés pour arrêter l'obturateur mobile uniquement à la première profondeur et à la deuxième profondeur pour la détection pré- et post-opératoire. On prévoira trois paires de crans, et donc trois paires d'organes d'arrêt, lorsque les moyens d'arrêt peuvent également arrêter l'obturateur mobile à la troisième position en peropératoire.

Bien entendu, on pourrait tout à fait prévoir une seule paire de crans, voire un seul cran, sur l'un parmi le corps et l'obturateur mobile, et deux ou trois organes d'arrêt ou paires d'organes d'arrêt, selon que l'on souhaite ou non pouvoir arrêter l'obturateur mobile à une troisième position, sur l'autre parmi le corps et l'obturateur mobile. Dans ce cas, ce sont les positions des organes d'arrêt le long du corps ou de l'obturateur mobile qui définiront les différentes profondeurs auxquelles l'obturateur mobile peut être arrêté. A l'inverse, on pourrait prévoir une seule paire d'organes d'arrêt, voire un seul organe d'arrêt, sur l'un parmi le corps et l'obturateur mobile, et deux ou trois crans ou paires de crans sur l'autre parmi le corps et l'obturateur mobile, et ce sont alors les positions des crans qui définiront les différentes profondeurs d'arrêt pour l'obturateur mobile.

La présente invention n'est pas limitée à une configuration particulière des organes d'arrêt. D'une manière générale, les organes d'arrêt constitueront des points de surélévation le long de l'ouverture latérale du corps ou de l'obturateur mobile, aptes à être engagés de manière sélective dans des crans. Les organes d'arrêt peuvent par exemple être chacun formés par une bille sollicitée élastiquement, notamment par ressort, vers une position d'engagement dans un cran. En variante, les organes d'arrêt pourront être chacun formés par un plot en matière déformable, de préférence élastiquement déformable, par exemple en silicone, pour permettre un engagement/désengagement automatique du plot dans/hors d'un cran par le seul déplacement de l'obturateur mobile. Un tel engagement/désengagement automatique est également obtenu avec des billes sollicitées élastiquement.

De manière avantageuse, l'ouverture latérale du corps peut s'étendre jusqu'à l'extrémité proximale du corps, la longueur de l'ouverture latérale, suivant l'axe longitudinal du corps, étant, de préférence, d'au moins 75 mm.

Comme indiqué ci-dessus, le dispositif selon la présente invention peut être dimensionné pour une utilisation peropératoire. Dans ce cas, le corps peut avoir un diamètre extérieur maximal de 28 mm. Un diamètre de 28 mm pour le corps permet au chirurgien d'utiliser ses doigts pour faire les noeuds des ligatures et ainsi de le dispenser d'un pousse-noeud qui est utilisé avec les dispositifs classiques.

Comme également indiqué ci-dessus, le dispositif selon la présente invention peut être dimensionné pour une utilisation préopératoire et post-opératoire. Dans ce cas, le corps peut avoir un diamètre extérieur maximal de 15 mm. Un corps d'un tel diamètre peut être introduit dans l'anus d'un patient sans requérir d'anesthésie, ce qui est particulièrement adapté pour une consultation préopératoire, pour l'évaluation de l'étendue de la maladie par le chirurgien, ou post-opératoire pour la surveillance de l'évolution de la maladie.

Avantageusement, l'obturateur mobile est transparent, ce qui facilite l'observation du canal anal par le chirurgien.

Avantageusement, le dispositif comprend en outre une sonde Doppler pour la détection des artères hémorroïdales, la sonde Doppler étant, de préférence, portée par l'obturateur mobile et dépassant de cette dernière, de préférence de 10 mm, pour s'étendre dans la fenêtre d'accès. Le fait que la sonde Doppler dépasse de l'obturateur mobile permet un meilleur contact et une meilleure visibilité de la muqueuse rectale.

De préférence, le dispositif comprend des moyens d'éclairage de la fenêtre d'accès, lesquels moyens d'éclairage comprennent, de préférence, une diode électroluminescente disposée à l'extrémité proximale du corps ou dans la paroi latérale du corps.

De préférence, les moyens de préhension sont formés par deux manettes diamétralement opposées et s'étendant perpendiculairement à l'extrémité proximale du corps. Ces deux manettes permettent au chirurgien de faire tourner le dispositif de 360° sans aucune difficulté.

Pour mieux illustrer l'objet de la présente invention, on va en décrire ci-après à titre indicatif et non limitatif un mode de réalisation particulier avec référence aux dessins annexés.

Sur ces dessins :
[Fig. 1] : vue en perspective du dispositif selon un mode de réalisation particulier de la présente invention ;
[Fig. 2] : vue du dispositif de la Figure 1, depuis l'extrémité proximale de ce dernier ;
[Fig. 3] : vue en perspective du corps du dispositif des Figures 1 et 2 ;
[Fig. 4] : vue en perspective de l'obturateur mobile du dispositif des Figures 1 et 2 ;
[Fig. 5] : vue en coupe du dispositif de la Figure 1, en cours d'intervention, à la première profondeur de l'obturateur mobile, pour les ligatures d'artères hémorroïdaires ;
[Fig. 6] : vue analogue à la Figure 1, le dispositif étant à la deuxième profondeur de l'obturateur mobile ; et
[Fig. 7] : vue analogue aux Figures 5 et 6, à la troisième profondeur de l'obturateur mobile, pour le traitement d'un prolapsus hémorroïdaire.

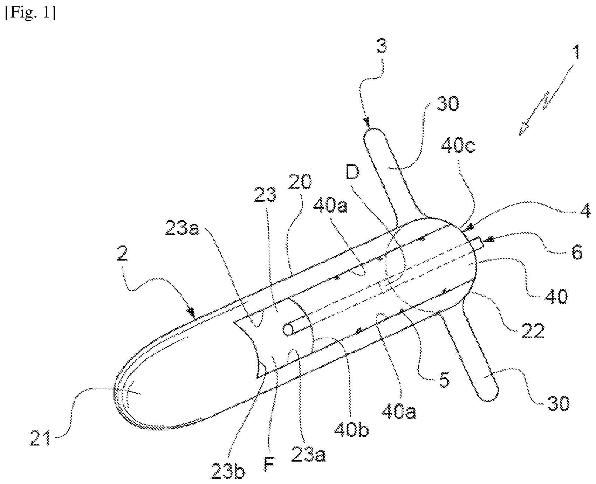

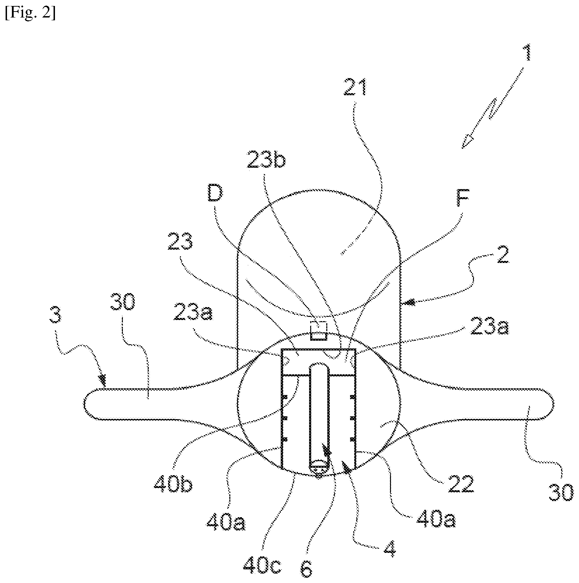

Si l'on se réfère tout d'abord aux Figures 1 et 2, on peut voir qu'un dispositif 1 selon un mode de réalisation particulier de la présente invention comprend d'une manière générale un corps 2, des moyens de préhension 3 et un obturateur mobile 4.

Le corps 2 est destiné à être introduit, au moins en partie, dans l'anus d'un patient et se présente, de manière classique, sous la forme d'une pièce tubulaire cylindrique 20 sur une majeure partie de sa longueur et dont l'extrémité distale 21 est arrondie de façon à être le moins traumatique possible et éviter les lésions du canal anal et du rectum. A cet effet, on pourra également prévoir de recouvrir de mousse l'extrémité distale 21.

Les moyens de préhension 3 consistent ici en deux manettes 30, par exemple se présentant simplement sous la forme de deux éléments longitudinaux plats s'étendant chacun à partir de l'extrémité proximale 22 ouverte du corps 2, sur des côtés opposés de cette dernière et en appartenant à un même diamètre. Ces manettes 30 peuvent être réalisées d'un seul tenant avec le corps 2 ou être fixées à celui-ci par tout moyen approprié, comme par exemple par soudure.

Le dispositif 1 selon la présente invention peut être à usage unique ou à usage multiple.

Dans le cas où le dispositif 1 est à usage unique, le corps 2, et le cas échéant également les manettes 30, peut par exemple être réalisé en un matériau composite, notamment de couleur blanche pour refléter la lumière dans le champ opératoire.

Dans le cas où le dispositif 1 est à usage multiple, le corps 2, et le cas échéant également les manettes 30, peut par exemple être réalisé en acier inoxydable.

Des moyens d'éclairage du champ opératoire seront avantageusement prévus, comme par exemple une diode électroluminescente D placée à l'extrémité proximale 22 du corps 2, comme illustré de manière schématique sur les Figures 1 et 2. Bien entendu, une telle diode électroluminescente pourra être prévue à un autre emplacement du dispositif, comme par exemple dans la paroi latérale du corps 2.

Comme indiqué ci-dessus, le diamètre extérieur de la pièce tubulaire 20 pourra, par exemple, être de 28 mm pour une utilisation peropératoire ou être de 15 mm pour une utilisation pré- ou post-opératoire.

Le corps 2 présente une ouverture 23 rectangulaire s'étendant à partir du bord circulaire de l'ouverture que forme l'extrémité distale 22, et s'achève au moins légèrement avant la région du corps 2 où ce dernier commence à ne plus être cylindrique. L'ouverture 23 présente ainsi une longueur dans la direction longitudinale du corps 2 et une largeur dans la direction circonférentielle du corps 2 (partie tubulaire 20).

L'ouverture 23 présente ainsi deux bords longitudinaux 23a et un bord transversal 23b. La largeur de l'ouverture 23 doit être suffisante pour permettre au chirurgien d'accéder à la paroi du bas rectum.

L'obturateur mobile 4 est monté dans l'ouverture 23 de façon à obturer en partie cette dernière. Une fenêtre d'accès F est ainsi délimitée entre le bord transversal 23b, les parties distales des deux bords longitudinaux 23a et le bord transversal distal 40b de l'obturateur mobile 4. Selon la présente invention, l'obturateur mobile 4 est monté déplaçable en translation le long du corps 2, de façon à faire varier la longueur de la fenêtre d'accès F sans déplacement du corps 2.

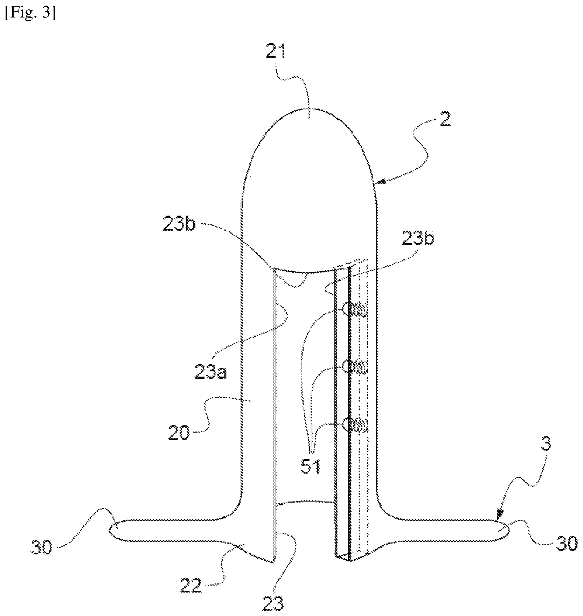

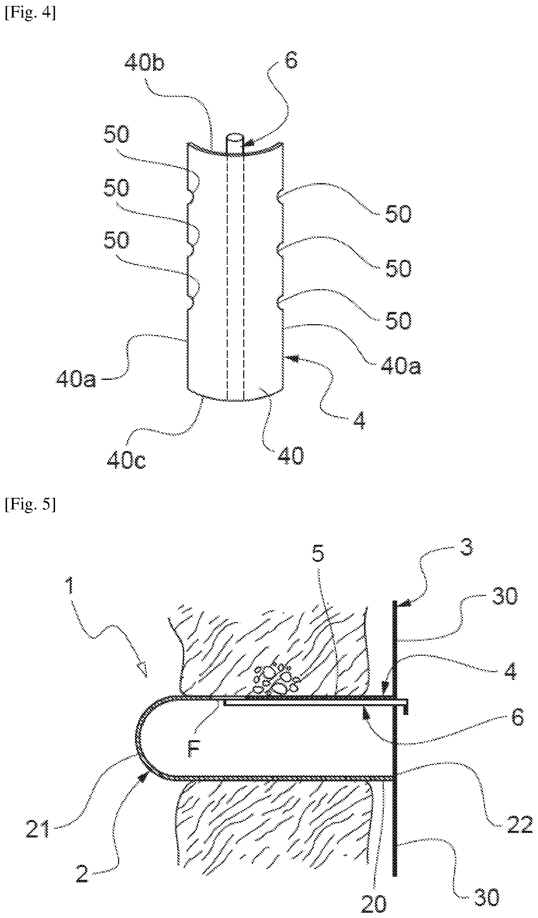

Dans un mode de réalisation particulier illustré sur les Figures 3 et 4, les deux bords longitudinaux 23a de l'ouverture 23 présentent chacun un profil en U dont l'ouverture est dirigée vers le bord longitudinal 23a opposé, de sorte que chaque bord longitudinal 23a forme une gorge. L'obturateur mobile 4 est quant à lui formé par une plaque incurvée 40 présentant la même courbure que la partie tubulaire 20 et dont les deux bords longitudinaux 40a sont chacun reçus dans une gorge respective, de sorte que l'obturateur mobile 4 est monté coulissant dans l'ouverture 23 avec guidage en translation par lesdites gorges.

La longueur de l'obturateur mobile 4 est inférieure à la longueur de l'ouverture 23. Ainsi, comme illustré sur les Figures 1 et 2, lorsque le bord transversal proximal 40c de l'obturateur mobile 4 se situe dans le plan de l'ouverture de l'extrémité distale 22 du corps 2, le bord transversal distal 40b de l'obturateur mobile 4 est à distance de celui de l'ouverture 23, de sorte que la fenêtre d'accès F est bien présente.

Comme illustré schématiquement, des moyens d'arrêt en translation 5 sont prévus pour permettre à l'obturateur mobile 4 d'être arrêté de manière sélective à trois profondeurs le long de l'ouverture 23.

Un exemple de tels moyens d'arrêt en translation 5 est illustré de manière plus précise sur les Figures 3 et 4, sur lesquelles on peut voir que les moyens d'arrêt en translation 5 comprennent trois paires de crans 50 sur les bords longitudinaux 40a de l'obturateur mobile 4 et trois paires d'organes d'arrêt 51 sur les bords longitudinaux 23a de l'ouverture latérale 23. Pour des raisons de lisibilité de la Figure 3, seuls les organes d'arrêt 51 sur l'un des deux bords longitudinaux 23a ont été illustrés.

L'espacement entre les trois crans 50 situés sur un même bord longitudinal 40a est égal à l'espacement entres les trois organes d'arrêt 51 situé sur un même bord longitudinal 23a.

Les organes d'arrêt 51 sont ici formés chacun par une bille sollicitée élastiquement par ressort 52 vers une position d'engagement, dans laquelle une partie de la bille fait saillie par rapport au bord longitudinal 23a de l'ouverture latérale 23 de façon à pouvoir être reçue dans un cran 50 lorsque ce dernier est placé en face de la bille. On prévoira ainsi sur le corps 2 des butées pour arrêter le mouvement des billes vers l'extérieur. Par exemple, chaque bille et ressort pourront être montés dans un logement ménagé à cet effet dans la paroi latérale du corps 2, ce logement débouchant sur le bord longitudinal 23a, notamment dans le fond des gorges dans lesquelles l'obturateur mobile 4 est monté coulissant, au niveau d'un orifice dont le diamètre est inférieur au diamètre de la bille, de sorte que la bille est empêchée de sortir dudit logement.

On souligne ici qu'aucune étanchéité n'est à prévoir en particulier, au niveau de ces organes d'arrêt 51.

Si l'on se réfère de nouveau aux Figures 1 et 2, on peut voir que l'obturateur mobile 4 porte une sonde Doppler 6, illustrée de manière schématique et bien connue en soi, de sorte que celle-ci puisse être mise en contact avec la muqueuse rectale. La sonde Doppler 6 pourra être à alimentation électrique filaire ou sans fil, comme par exemple par Wi-Fi, et sera reliée à des moyens de traitement du signal de détection et des moyens d'affichage également bien connus et déjà utilisés dans la technique HAL-RAR Doppler.

La sonde Doppler 6 pourra être rendue solidaire de l'obturateur mobile 4, notamment sur la face interne de ce dernier, par tout moyen approprié, comme par exemple par collage ou par un système de rails de maintien faisant partie de la pièce mobile 4.

La sonde Doppler 6 pourra être à usage unique ou usage multiple.

On pourrait également prévoir qu'elle apporte de la lumière dans le champ opératoire par un éclairage incorporé à son extrémité distale.

Selon la présente invention, la sonde Doppler 6 fera avantageusement saillie dans la fenêtre d'accès F, de préférence sur 10 mm, pour un meilleur contact et une meilleure visibilité de la muqueuse rectale au lieu du contact.

L'obturateur mobile 4 est avantageusement transparent, de façon à permettre au chirurgien de voir la zone de détection par la sonde Doppler 6 et de voir s'il y a ou non d'autres lésions du bas rectum, telles que des polypes ou des ulcères par exemple.

De préférence, le dispositif selon la présente invention présentera les dimensions suivantes :
- longueur totale du dispositif : 90 mm (de la base des manettes 30 jusqu'à l'extrémité distale 21 du corps 2) ;
- manettes 30 : épaisseur de 5 mm et longueur de 30 mm ;
- corps 2 : longueur de la partie tubulaire 20 est de 70 mm, longueur de la partie distale arrondie est de 20 mm ;
- ouverture 23 : longueur de 75 mm ;
- diamètre extérieur du corps : 28 mm pour intervention chirurgicale et 15 mm pour consultations pré- et postopératoires ; et
- obturateur mobile 4 : longueur de 55 mm, les trois paires de crans sont situées à 10, 20 et 30 mm du bord transversal distal 40b.

On va maintenant décrire succinctement le principe de fonctionnement du dispositif 1 en utilisation peropératoire, avec référence aux Figures 5 à 7.

Sur la Figure 5, on a représenté le dispositif 1 après son introduction dans le canal anal d'un patient, jusqu'à ce que les manettes 30 viennent au contact du patient.

L'obturateur mobile 4 est alors à la première profondeur et le chirurgien fait tourner lentement le dispositif 1 jusqu'à détection de la première artère hémorroïdaire. La rotation du dispositif 1 est facilitée par les deux manettes 30.

A la détection de la première artère hémorroïdaire, le chirurgien peut procéder à la ligature de cette dernière, via la fenêtre d'accès F.

Le chirurgien procède à un tour complet du dispositif 1, comme dans l'état antérieur de la technique.

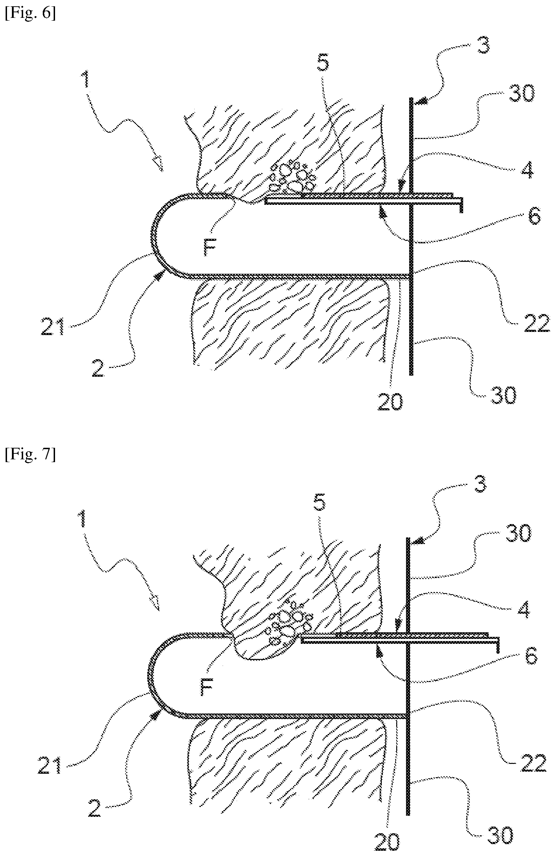

Une fois que ce premier tour complet a été effectué, le chirurgien déplace l'obturateur mobile 4, et seulement celui-ci, en le tirant vers l'extérieur, jusqu'à la deuxième profondeur, comme illustré sur la Figure 6.

Le chirurgien peut alors procéder au second tour complet de détection des artères hémorroïdaires et, le cas échéant, procéder aux ligatures de celles-ci.

Après le second tour complet de détection, le chirurgien tire de nouveau l'obturateur mobile 4 vers l'extérieur, jusqu'à la troisième profondeur, comme illustré sur la Figure 7. Dans cette position, la fenêtre d'accès F est suffisante pour le traitement d'éventuels prolapsus hémorroïdaires, sans que l'obturateur mobile 4 n'entrave les gestes du chirurgien.

On constate donc que le dispositif 1 selon le mode de réalisation particulier de la présente invention permet de conduire les trois étapes du traitement que sont la détection des artères hémorroïdaires, la ligature de celles-ci et la mucopexie des éventuels prolapsus hémorroïdaires, sans déplacement du corps le long du canal anal après que la première artère hémorroïdaire a été détectée, et ceci de manière aisée et sans risque de non-détection d'une artère hémorroïdaire.

Ce geste peut être fait de façon précise sur deux étages du bas rectum, ce qu'aucun autre dispositif ne permet actuellement, et par conséquent, de réduire considérablement la possibilité de récidive de la maladie hémorroïdaire.

## Revendications

1. Dispositif (1) pour le traitement de la maladie hémorroïdaire, comprenant :
- un corps (2) tubulaire cylindrique destiné à être partiellement introduit dans l'anus d'un patient, l'extrémité distale (21) du corps (2) étant fermée tandis que son extrémité proximale (22) est ouverte pour autoriser un accès à l'intérieur du corps (2), une ouverture latérale (23) étant ménagée dans la paroi latérale du corps de façon à former une fenêtre d'accès (F) au bas rectum,
- des moyens de préhension (3) s'étendant à partir de l'extrémité proximale (22) du corps (2), pour permettre la manipulation du corps (2) depuis l'extérieur une fois le corps (2) introduit dans l'anus du patient,
- un obturateur mobile (4) obturant en partie l'ouverture latérale (23) du corps (2) de façon à délimiter avec cette dernière la fenêtre d'accès (F), l'obturateur mobile (4) étant déplaçable en translation le long du corps (2), suivant l'axe longitudinal de ce dernier, de façon à faire varier la longueur de la fenêtre d'accès (F), et
- des moyens (5) d'arrêt en translation de l'obturateur mobile (4) par rapport au corps (2), aptes à arrêter de manière sélective l'obturateur mobile (4) au moins à une première profondeur et une deuxième profondeur le long du corps (2), **caractérisé par le fait que**
la première profondeur étant la position où l'obturateur mobile (4) est le plus proche de l'extrémité distale (21) du corps (2) et où ladite fenêtre d'accès (F) permet un accès aux artères au niveau du bas du rectum,
et les moyens d'arrêt en translation (5) sont également aptes à arrêter de manière sélective l'obturateur mobile (4) à une troisième profondeur, à distance de la deuxième profondeur suivant l'axe longitudinal du corps (2).

2. Dispositif (1) selon la revendication 1, **caractérisé par le fait que** la deuxième profondeur est à 10 mm, suivant l'axe longitudinal du corps (2), de la première profondeur.

3. Dispositif (1) selon l'une des revendications 1 et 2, **caractérisé par le fait que** la troisième profondeur est à 10 mm de la deuxième profondeur.

4. Dispositif (1) selon l'une quelconque des revendications 1 à 3, **caractérisé par le fait qu'**à la première profondeur, l'extrémité distale de l'obturateur mobile (4) est à une distance, suivant l'axe longitudinal du corps (2), de 55 mm de l'extrémité proximale (22) du corps (2).

5. Dispositif (1) selon l'une quelconque des revendications 1 à 4, **caractérisé par le fait que** l'obturateur mobile (4) est monté coulissant dans l'ouverture latérale (23) du corps (2).

6. Dispositif (1) selon la revendication 5, **caractérisé par le fait que** les moyens d'arrêt en translation (5) comprennent, d'une part, des crans (50) ménagés sur l'un parmi des bords longitudinaux (23a) de l'ouverture latérale (23) du corps (2) et des bords longitudinaux (40a) de l'obturateur mobile (4), et, d'autre part, des organes d'arrêt (51) prévus sur l'autre parmi les bords longitudinaux (23a) de l'ouverture latérale (23) du corps (2) et les bords longitudinaux (40a) de l'obturateur mobile (4), les crans (50) et les organes d'arrêt (51) étant positionnés pour qu'à chacune des première, deuxième et troisième profondeurs, au moins un organe d'arrêt (51) est engagé dans un cran (50).

7. Dispositif (1) selon la revendication 6, **caractérisé par le fait que** l'obturateur mobile (4) comprend deux paires de crans (50), de préférence trois paires de crans (50), chaque cran (50) d'une même paire étant situé sur un bord longitudinal (40a) respectif de l'obturateur mobile (4), avec de préférence un espacement de 10 mm, suivant l'axe longitudinal du corps (2), entre deux paires de crans (50) successives, et le corps (2) comprend des paires d'organes d'arrêt (51) en même nombre que les paires de crans (50), chaque organe d'arrêt (51) d'une même paire étant situé sur un bord longitudinal (23a) respectif de l'ouverture latérale (23), et l'espacement entre deux paires d'organes d'arrêt (51) successives étant égal à l'espacement entre deux paires de crans (50) successives.

8. Dispositif (1) selon l'une quelconque des revendications 1 à 7, **caractérisé par le fait que** l'ouverture latérale (23) du corps (2) s'étend jusqu'à l'extrémité proximale (22) du corps (2), la longueur de l'ouverture latérale (23), suivant l'axe longitudinal du corps (2), étant, de préférence, d'au moins 75 mm.

9. Dispositif (1) selon l'une quelconque des revendications 1 à 8, **caractérisé par le fait que** le corps (2) a un diamètre extérieur maximal de 28 mm.

10. Dispositif (1) selon l'une quelconque des revendications 1 à 8, **caractérisé par le fait que** le corps (2) a un diamètre extérieur maximal de 15 mm.

11. Dispositif (1) selon l'une quelconque des revendications 1 à 10, **caractérisé par le fait que** l'obturateur mobile (4) est transparent.

12. Dispositif (1) selon l'une quelconque des revendications 1 à 11, **caractérisé par le fait qu'**il comprend en outre une sonde Doppler (6) pour la détection des artères hémorroïdales, la sonde Doppler (6) étant, de préférence, portée par l'obturateur mobile (4) et dépassant de cette dernière, de préférence de 10 mm, pour s'étendre dans la fenêtre d'accès (F).

13. Dispositif (1) selon l'une quelconque des revendications 1 à 12, **caractérisé par le fait qu'**il comprend des moyens d'éclairage de la fenêtre d'accès (F), lesquels moyens d'éclairage comprennent, de préférence, une diode électroluminescente (D) disposée à l'extrémité proximale (22) du corps (2) ou dans la paroi latérale du corps (2).

14. Dispositif (1) selon l'une quelconque des revendications 1 à 13, **caractérisé par le fait que** les moyens de préhension (3) sont formés par deux manettes (30) diamétralement opposées et s'étendant perpendiculairement à l'extrémité proximale (22) du corps (2).

## Patentansprüche

1. - Vorrichtung (1) für die Behandlung der Hämorrhoidenerkrankung, umfassend:
- einen rohrförmigen zylindrischen Körper (2), der dazu bestimmt ist, teilweise in den Anus eines Patienten eingeführt zu werden, wobei das distale Ende (21) des Körpers (2) verschlossen ist, während das proximale Ende (22) offen ist, um einen Zugang zum Inneren des Körpers (2) zu ermöglichen, wobei eine seitliche Öffnung (23) in der Seitenwand des Körpers ausgebildet ist, um ein Zugangsfenster (F) zu dem unteren Rektum zu bilden,
- Greifeinrichtungen (3), die sich von dem proximalen Ende (22) des Körpers (2) erstrecken, um eine Manipulation des Körpers (2) von außen zu ermöglichen, nachdem der Körper (2) in den Anus des Patienten eingeführt wurde,
- einen beweglichen Verschluss (4), der die seitliche Öffnung (23) des Körpers (2) teilweise verschließt, um mit dieser letzteren das Zugangsfenster (F) zu begrenzen, wobei der bewegliche Verschluss (4) entlang des Körpers (2) entlang dessen Längsachse translatorisch verschiebbar ist, um die Länge des Zugangsfensters (F) zu variieren, und
- Translationsarretiereinrichtungen (5) des beweglichen Verschlusses (4) in Bezug auf den Körper (2), die geeignet sind, um den beweglichen Verschluss (4) selektiv zumindest in einer ersten Tiefe und einer zweiten Tiefe entlang des Körpers (2) zu arretieren,
**dadurch gekennzeichnet, dass** die erste Tiefe die Position ist, in der der bewegliche Verschluss (4) dem distalen Ende (21) des Körpers (2) am nächsten ist und in der das Zugangsfenster (F) einen Zugang zu den Arterien auf Höhe des unteren Rektums ermöglicht, und die Translationsarretiereinrichtungen (5) auch geeignet sind, den beweglichen Verschluss (4) selektiv bei einer dritten Tiefe zu arretieren, die entlang der Längsachse des Körpers (2) von der zweiten Tiefe beabstandet ist.

2. - Vorrichtung (1) nach Anspruch 1, **dadurch gekennzeichnet, dass** die zweite Tiefe entlang der Längsachse des Körpers (2) 10 mm von der ersten Tiefe entfernt ist.

3. - Vorrichtung (1) nach einem der Ansprüche 1 oder 2, **dadurch gekennzeichnet, dass** die dritte Tiefe 10 mm von der zweiten Tiefe entfernt ist.

4. - Vorrichtung (1) nach einem der Ansprüche 1 bis 3, **dadurch gekennzeichnet, dass** das distale Ende des beweglichen Verschlusses (4) bei der ersten Tiefe in einem Abstand von 55 mm entlang der Längsachse des Körpers (2) von dem proximalen Ende (22) des Körpers (2) entfernt ist.

5. - Vorrichtung (1) nach einem der Ansprüche 1 bis 4, **dadurch gekennzeichnet, dass** der bewegliche Verschluss (4) verschiebbar in der seitlichen Öffnung (23) des Körpers (2) montiert ist.

6. - Vorrichtung (1) nach Anspruch 5, **dadurch gekennzeichnet, dass** die Translationsarretiereinrichtungen (5) einerseits Rasten (50) umfassen, die an einem der Längsränder (23a) der Seitenöffnung (23) des Körpers (2) und der Längsränder (40a) des beweglichen Verschlusses (4) ausgebildet sind, und andererseits, Arretierelemente (51), die an dem anderen von den Längsrändern (23a) der seitlichen Öffnung (23) des Körpers (2) und den Längsrändern (40a) des beweglichen Verschlusses (4) bereitgestellt sind, wobei die Rasten (50) und die Arretierelemente (51) positioniert sind, sodass bei jeder von der ersten, der zweiten und der dritten Tiefe mindestens ein Arretierelement (51) mit einer Raste (50) in Eingriff ist.

7. - Vorrichtung (1) nach Anspruch 6, **dadurch gekennzeichnet, dass** der bewegliche Verschluss (4) zwei Paare von Rasten (50), vorzugsweise drei Paare von Rasten (50), umfasst, wobei jede Raste (50) ein und desselben Paars an einer jeweiligen Längskante (40a) des beweglichen Verschlusses (4) angeordnet ist, wobei vorzugsweise ein Abstand von 10 mm entlang der Längsachse des Körpers (2) zwischen zwei aufeinanderfolgenden Paaren von Rasten (50) vorhanden ist, und der Körper (2) Paare von Arretierelementen (51) in der gleichen Anzahl wie die Paare von Rasten (50) umfasst, wobei jedes Arretierelement (51) eines gleichen Paars an einer jeweiligen Längskante (23a) der Seitenöffnung (23) angeordnet ist und der Abstand zwischen zwei aufeinanderfolgenden Paaren von Arretierelementen (51) gleich ist wie der Abstand zwischen zwei aufeinanderfolgenden Paaren von Rasten (50).

8. - Vorrichtung (1) nach einem der Ansprüche 1 bis 7, **dadurch gekennzeichnet, dass** sich die seitliche Öffnung (23) des Körpers (2) bis zu dem proximalen Ende (22) des Körpers (2) erstreckt, wobei die Länge der seitlichen Öffnung (23), entlang der Längsachse des Körpers (2) vorzugsweise mindestens 75 mm ist.

9. - Vorrichtung (1) nach einem der Ansprüche 1 bis 8, **dadurch gekennzeichnet, dass** der Körper (2) einen maximalen Außendurchmesser von 28 mm aufweist.

10. - Vorrichtung (1) nach einem der Ansprüche 1 bis 8, **dadurch gekennzeichnet, dass** der Körper (2) einen maximalen Außendurchmesser von 15 mm aufweist.

11. - Vorrichtung (1) nach einem der Ansprüche 1 bis 10, **dadurch gekennzeichnet, dass** der bewegliche Verschluss (4) transparent ist.

12. - Vorrichtung (1) nach einem der Ansprüche 1 bis 11, **dadurch gekennzeichnet, dass** sie außerdem eine Dopplersonde (6) zur Erfassung von Hämorrhoidalarterien umfasst, wobei die Dopplersonde (6) vorzugsweise von dem beweglichen Verschluss (4) getragen wird und von diesem letzteren vorzugsweise 10 mm hervorsteht, um sich in das Zugangsfenster (F) zu erstrecken.

13. - Vorrichtung (1) nach einem der Ansprüche 1 bis 12, **dadurch gekennzeichnet, dass** sie Beleuchtungseinrichtungen des Zugangsfensters (F) umfasst, wobei diese Beleuchtungseinrichtungen vorzugsweise eine lichtemittierende Diode (D) umfassen, die an dem proximalen Ende (22) des Körpers (2) oder in der Seitenwand des Körpers (2) angeordnet ist.

14. - Vorrichtung (1) nach einem der Ansprüche 1 bis 13, **dadurch gekennzeichnet, dass** die Greifeinrichtungen (3) durch zwei diametral gegenüberliegende und sich senkrecht zu dem proximalen Ende (22) des Körpers (2) erstreckende Griffe (30) gebildet sind.

## Claims

1. - A device (1) for the treatment of haemorrhoidal disease, comprising:
- a cylindrical tubular body (2) intended to be partially introduced into the anus of a patient, the distal end (21) of the body (2) being closed while its proximal end (22) is open to allow access to the inside of the body (2), a side opening (23) being provided in the side wall of the body so as to form an access window (F) to access the lower rectum,
- gripping means (3) extending from the proximal end (22) of the body (2), to allow manipulation of the body (2) from the outside once the body (2) has been introduced into the patient's anus,
- a movable shutter (4) partially closing off the side opening (23) of the body (2) so as to delimit with the latter the access window (F), the movable shutter (4) being movable in translation along the body (2), along the longitudinal axis of the latter, so as to vary the length of the access window (F), and
- means (5) for stopping in translation the movable shutter (4) relative to the body (2), capable of selectively stopping the movable shutter (4) at least at a first depth and a second depth along the body (2),
**characterised in that** the first depth being the position where the movable shutter (4) is closest to the distal end (21) of the body (2) and where said access window (F) allows access to the arteries at the lower rectum, and the means for stopping in translation (5) are also capable of selectively stopping the movable shutter (4) at a third depth, at a distance from the second depth along the longitudinal axis of the body (2).

2. - The device (1) according to claim 1, **characterised in that** the second depth is 10 mm, along the longitudinal axis of the body (2), from the first depth.

3. - The device (1) according to one of claims 1 and 2, **characterised in that** the third depth is 10 mm from the second depth.

4. - The device (1) according to any one of claims 1 to 3, **characterised in that** at the first depth, the distal end of the movable shutter (4) is at a distance, along the longitudinal axis of the body (2), of 55 mm from the proximal end (22) of the body (2).

5. - The device (1) according to any one of claims 1 to 4, **characterised in that** the movable shutter (4) is slidably mounted in the side opening (23) of the body (2).

6. - The device (1) according to claim 5, **characterised in that** the means for stopping in translation (5) comprise, on the one hand, notches (50) formed on one among longitudinal edges (23a) of the side opening (23) of the body (2) and longitudinal edges (40a) of the movable shutter (4), and, on the other hand, stop members (51) provided on the other among the longitudinal edges (23a) of the side opening (23) of the body (2) and the longitudinal edges (40a) of the movable shutter (4), the notches (50) and the stop members (51) being positioned so that at each of the first, second and third depths, at least one stop member (51) is engaged in a notch (50).

7. - The device (1) according to claim 6, **characterised in that** the movable shutter (4) comprises two pairs of notches (50), preferably three pairs of notches (50), each notch (50) of the same pair being located on a respective longitudinal edge (40a) of the movable shutter (4), preferably with a spacing of 10 mm, along the longitudinal axis of the body (2), between two pairs of successive notches (50), and the body (2) comprises pairs of stop members (51) in the same number as the pairs of notches (50), each stop member (51) of the same pair being located on a respective longitudinal edge (23a) of the side opening (23), and the spacing between two successive pairs of stop members (51) being equal to the spacing between two successive pairs of notches (50).

8. - The device (1) according to any one of claims 1 to 7, **characterised in that** the side opening (23) of the body (2) extends to the proximal end (22) of the body (2), the length of the side opening (23), along the longitudinal axis of the body (2), preferably being at least 75 mm.

9. - The device (1) according to any one of claims 1 to 8, **characterised in that** the body (2) has a maximum outer diameter of 28 mm.

10. - The device (1) according to any one of claims 1 to 8, **characterised in that** the body (2) has a maximum outer diameter of 15 mm.

11. - The device (1) according to any one of claims 1 to 10, **characterised in that** the movable shutter (4) is transparent.

12. - The device (1) according to any one of claims 1 to 11, **characterised in that** it further comprises a Doppler probe (6) for detecting haemorrhoidal arteries, the Doppler probe (6) preferably being carried by the movable shutter (4) and projecting therefrom, preferably by 10 mm, to extend into the access window (F).

13. - The device (1) according to any one of claims 1 to 12, **characterised in that** it comprises means for illuminating the access window (F), which illuminating means preferably comprise a light-emitting diode (D) arranged at the proximal end (22) of the body (2) or in the side wall of the body (2).

14. - The device (1) according to any one of claims 1 to 13, **characterised in that** the gripping means (3) are formed by two diametrically opposed handles (30) extending perpendicularly to the proximal end (22) of the body (2).
